# EUROPÄISCHE PATENTSCHRIFT

(11) **EP 0 755 908 B1**
(45) Veröffentlichungstag und Bekanntmachung des Hinweises auf die Patenterteilung: **12.01.2000**
(21) Anmeldenummer: 96109684.9
(22) Anmeldetag: 17.06.1996
(51) Int. Cl.: C07C 17/16, C07C 25/02

(54) **Verfahren zur Herstellung von substituierten Chloraromaten**
Process for preparing substituted chloro-aromatic compounds
Procédé pour la préparation des composés aromatiques chlorés substitués

(30) Priorität: 29.06.1995 DE 19523641
(43) Veröffentlichungstag der Anmeldung: 29.01.1997
(73) Patentinhaber: BAYER AG, 51368 Leverkusen (DE)
(72) Erfinder: Landscheidt, Heinz, Dr., 47057 Duisburg (DE); Broda, Witold, Dr., 53819 Neunkirchen-Seelscheid (DE); Klausener, Alexander, Dr., 50670 Köln (DE)

(56) Entgegenhaltungen:
- EP-A- 0 223 421
- EP-A- 0 514 683
- Römpp: Lexikon Chemie, 10. Aufl., 1996: "Alkohole", "Phenole", S. 112-114 und 3250-3251

## Beschreibung

Die vorliegende Erfindung betrifft ein Verfahren zur Herstellung substituierter Chloraromaten durch Umsetzung von substituierten Phenolen mit Triphenylphosphindichlorid.

Chloraromaten der verschiedensten Substitutionsmuster werden zu den verschiedensten Verwendungszwecken benötigt, beispielsweise als Vorprodukte zur Herstellung von Pflanzenschutzmitteln und Pharmazeutika (siehe Ullmann, Encyclopädie der technischen Chemie, 4. Auflage, Seite 499 ff.).

Grundsätzlich kann man substituierte Chloraromaten durch direkte Chlorierung substituierter aromatischer Kohlenwasserstoffe herstellen. Hierbei bilden sich jedoch stets auch unerwünschte Isomere, so daß entweder eine geringe Produktqualität in Kauf genommen werden muß oder isomerenreine Verbindungen erst nach der Durchführung aufwendiger Trennoperationen erhalten werden können.

Ein weiteres Verfahren zur Herstellung von Chloraromaten umfaßt die Nitrierung von Aromaten, anschließende Reduktion der Nitroverbindung, Diazotierung des so erhaltenen Amins und Zersetzung des dabei gebildeten Diazoniumsalzes in Gegenwart von Kupferchlorid. Auch hierbei bilden sich im ersten Schritt unerwünschte Isomere. Außerdem ist eine derartige mehrstufige Synthesesequenz aufgrund des großen Aufwandes an Chemikalien und Arbeitskapazität unter wirtschaftlichen Gesichtspunkten ohne Interesse.

Vorwiegend im Bereich der heterocyclischen Verbindungen beschriebene Methoden erlauben einen selektiven Austausch einer Hydroxylgruppe gegen einen Chlorsubstituenten. So wird in Org. Synth. Coll. Vol. 3, S. 272 die Umsetzung von 4-Hydroxychinolin mit Phosphoroxychlorid, in J. Am. Chem. Soc. 76, 1109 (1954) die Umsetzung von 4,10-Dihydroxy-1,7-phenanthrolin mit Phosphorpentachlorid und in J. Org. Chem. 27, 1462 (1962) die Umsetzung eines Hydroxypyrimidin-Derivates mit Phosphoroxychlorid beschrieben. Gemäß J. Chem. Soc. 1964, 1666 lassen sich Chloropteridine durch Umsetzung entsprechender Hydroxyverbindungen mit Phosphorpentachlorid erhalten und gemäß J. Chem. Soc. 1944, 678 Chlorpyrimidine aus den entsprechenden Hydroxyverbindungen und Phosphoroxychlorid. Die genannten Verfahren beschränken sich auf die Umwandlung spezieller Heterocyclen. Sie sind offenbar weder auf andere Heterocyclen, noch auf carbocyclische Systeme übertragbar. Außerdem werden bei den genannten Verfahren neben den gewünschten Chlorheterocyclen die Umsetzungsprodukte der zum Einsatz gekommenen anorganischen Phosphorverbindungen in einer nicht regenerierbaren Form erhalten, so daß im Falle einer technischen Nutzung eine aufwendige Entsorgung für diese Phosphorverbindungen erforderlich ist.

In Lieb. Ann. Chem. 698, 106 (1966) wird die Zersetzung eines aus Phenol erhaltenen Chlorameisensäureesters mit Triphenylphosphin beschrieben, wobei als Reaktionsprodukt Chlorbenzol erhalten wird. Hierbei muß der Chlorameisensäureester in aufwendiger Weise hergestellt werden. Es ist außerdem nicht ersichtlich, ob sich auch substituierte Phenole nach diesem Verfahren zu substituierten Chloraromaten umsetzen lassen.

Es besteht daher das Bedürfnis nach einem Verfahren, das es ermöglicht, substituierte Chloraromaten auf einfache Weise aus leicht zugänglichen Ausgangsstoffen herzustellen.

Es wurde nun ein Verfahren zur Herstellung von substituierten Chloraromaten der Formel (I) gefunden 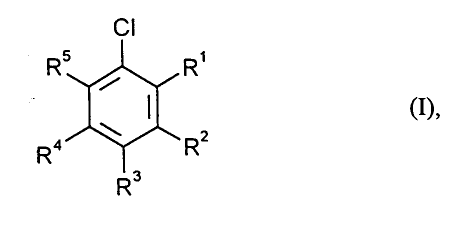 in der
- R¹ bis R⁵: unabhängig voneinander jeweils Wasserstoff, C₁-C₁₀-Alkyl, gegebenenfalls substituiertes C₆-C₁₀-Aryl oder Halogen bedeuten, wobei mindestens einer dieser Reste von Wasserstoff verschieden ist,
das dadurch gekennzeichnet ist, daß man substituierte Phenole der Formel (II) 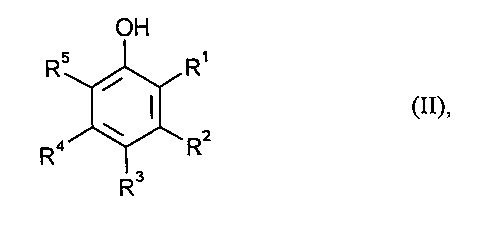 in der
- R¹ bis R⁵: die bei Formel (I) angegebene Bedeutung haben,
mit Triphenylphosphindichlorid umsetzt.

Soweit R¹ bis R⁵ für C₁-C₁₀-Alkyl stehen ist geradkettiges und verzweigtes C₁-C₆-Alkyl, insbesondere C₁-C₄-Alkyl bevorzugt. Besonders bevorzugt ist Methyl.

Soweit R¹ bis R⁵ für gegebenenfalls substituiertes C₆-C₁₀-Aryl stehen ist Phenyl und Naphthyl bevorzugt, wobei als Substituenten z.B. C₁-C₄-Alkyl und Halogen in Frage kommen. Besonders bevorzugt ist gegebenenfalls substituiertes Phenyl, wobei als Substituenten insbesondere Methyl, Fluor und Chlor in Frage kommen. Ganz besonders bevorzugt ist Phenyl.

Soweit R¹ bis R⁵ für Halogen stehen kann es sich z.B. um Fluor, Chlor und Brom handeln. Bevorzugt sind Fluor und Chlor, insbesondere Chlor.

Besonders bevorzugte Einzelverbindungen der Formel (II) sind Monochlorphenol, Monochlorkresole, Dichlorphenole und Dichlorkresole. Entsprechend werden erfindungsgemäß besonders bevorzugt Dichlorbenzole, Dichlortoluole, Trichlorbenzole und Trichlortoluole hergestellt.

Chlorhaltige Verbindungen der Formel (II) kann man nicht nur in reiner Form, sondern auch in Form von Reaktionsgemischen einsetzen, wie sie z.B. bei der Chlorierung von solchen Phenolen der Formel (II) erhalten werden, bei denen mindestens 2 und höchstens 4 der Reste R¹ bis R⁵ für Wasserstoff stehen.

Ein besonders vorteilhaftes Verfahren zur Herstellung von substituierten Chloraromaten der Formel (I), in der wenigstens einer der Reste R¹ bis R⁵ für Chlor steht ist deshalb dadurch gekennzeichnet, daß man zunächst ein Phenol der Formel (II), in der mindestens 2 und höchstens 4 der Reste R¹ bis R⁵ für Wasserstoff stehen, auf übliche Weise chloriert, z.B. mit Chlor in Gegenwart eines Lösungsmittels, und dann das erhaltene Reaktionsgemisch mit Triphenylphosphindichlorid umsetzt.

Triphenylphosphindichlorid kann in das erfindungsgemäße Verfahren als solches eingesetzt werden. Es ist jedoch bevorzugt, das Triphenylphosphindichlorid in situ zu erzeugen. Diese in situ-Erzeugung kann z.B. erfolgen, indem man Triphenylphosphin mit Chlor oder Triphenylphosphinoxid mit Phosgen in einem inerten Lösungsmittel umsetzt (siehe z.B. Z. Anorg. Chem. 250, 257 (1943) und Gazz. Chim. Ital 77, 509 (1947)) und das dabei erhältliche Reaktionsgemisch in das erfindungsgemäße Verfahren einsetzt. Es ist überraschend, daß sich in situ hergestelltes Triphenylphosphindichlorid ohne wesentliche Beeinträchtigung des Reaktionsverlaufes hinsichtlich Reaktionsgeschwindigkeit und Selektivität verwenden läßt.

Das erfindungsgemäße Verfahren wird vorzugsweise in Gegenwart eines inerten Lösungsmittels durchgeführt. Geeignet sind beispielsweise Halogenaromaten wie Chlorbenzol, Dichlorbenzole und Trichlorbenzole. Inerte Lösungsmittel kann man z.B. in Mengen von 0,1 bis 100 l pro Mol eingesetztes Triphenylphosphindichlorid anwenden. Wenn man das Triphenylphosphindichlorid in situ herstellt, kann das inerte Lösungsmittel bereits ganz oder teilweise in diese in situ-Herstellung eingesetzt werden.

Man kann das erfindungsgemäße Verfahren z.B. so durchführen, daß man zu Triphenylphosphindichlorid die zu chlorierende Substanz der allgemeinen Formel (II) hinzufügt, z.B. in einem Molverhältnis von 0,5 bis 10, vorzugsweise von 0,8 bis 5, bezogen auf das vorgelegte Triphenylphosphindichlorid. Die Verbindung der Formel (II) kann z.B. als solche, im Falle chlorhaltiger Verbindungen gegebenenfalls wie oben beschrieben als aus der Chlorierung kommendes Reaktionsgemisch oder vermischt mit einem inerten Lösungsmittel, z.B. der oben beschriebenen Art, zugegeben werden. Das Zusammenfügen der Reaktanten kann z.B. bei Temperaturen im Bereich 0 bis 200°C erfolgen.

Die erfindungsgemäße Umsetzung kann z.B. bei Temperaturen im Bereich von 50 bis 250°C durchgeführt werden. Die Reaktion ist dann beendet, wenn die Entwicklung von Chlorwasserstoffgas aufhört.

Nach Beendigung der Umsetzung kann man das dann vorliegende Reaktionsgemisch beispielsweise so aufarbeiten, daß man zunächst bei Normaldruck oder vermindertem Druck das inerte Lösungsmittel und das hergestellte Produkt der Formel (I) abdestilliert. Das so abgetrennte Produkt der Formel (I) kann gegebenenfalls durch weitere nachgeschaltete Schritte weiter gereinigt werden, beispielsweise durch Destillation, Kristallisation und andere dem Fachmann an sich bekannte Verfahren.

Der nach der Abtrennung des Lösungsmittels und des Produkts der Formel (I) verbleibende Rückstand besteht überwiegend aus Triphenylphosphinoxid, das wieder in Triphenylphosphindichlorid umgewandelt werden kann. Beispielsweise kann man den Rückstand in einem inerten Lösungsmittel, z.B. der obengenannten Art aufnehmen und durch die Einwirkung von Phosgen wieder in Triphenylphosphindichlorid überführen. So erhaltenes Triphenylphosphindichlorid kann erneut für Umsetzungen mit einer Verbindung der Formel (I) eingesetzt werden.

Das erfindungsgemäße Verfahren hat eine Reihe von Vorteilen. So gestattet es die Herstellung von substituierten Chloraromaten aus gut zugänglichen Ausgangsstoffen in einem auf einfache Weise durchzuführenden Verfahren, wobei die substituierten Chloraromaten in guten Ausbeuten erhalten werden. Wesentlich ist, daß man so praktisch isomerenreine substituierte Chloraromaten erhalten kann, wie sie für die weitere Verarbeitung zu beispielsweise Pflanzenschutzmittel und Pharmazeutika gefragt sind.

### Beispiele

### Beispiel 1

Zu einer Mischung aus 880 g Triphenylphosphin und 1000 ml Chlorbenzol wurden unter intensivem Rühren innerhalb von 3 Stunden 239 g Chlor eingeleitet, wobei die Temperatur während der ersten 2 Stunden 40°C, danach 50°C betrug. Zu dem so erhaltenen Gemisch ließ man im Verlauf von 90 Minuten bei 40°C eine Lösung von 456 g 2-Chlor-3-methyl-phenol in 200 ml Chlorbenzol zutropfen. Anschließend wurde die Temperatur des Reaktionsgemisches bei Normaldruck nach und nach auf bis zu 150°C erhöht, wobei beim Erreichen von 80°C eine intensive Entwicklung von gasförmigem Chlorwasserstoff eintrat. Noch während der Reaktion und anschließend daran wurden bis zum Erreichen einer Sumpftemperatur von 150°C insgesamt 1314 g Chlorwasserstoff enthaltendes Chlorbenzol als Destillat aufgefangen. Der verbleibende Rückstand wurde einer Destillation bei 5 bis 10 mbar unterworfen. So wurde 2,3-Dichlortoluol in einer Ausbeute von 92 % der Theorie erhalten.

### Beispiel 2

a) Herstellung von 2,4-Dichlor-6-methyl-phenol (Reaktionsgemisch)
   In eine Lösung aus 108 g o-Kresol in 500 ml Chlorbenzol wurden unter Eiskühlung innerhalb von 2 Stunden 142 g Chlor eingeleitet.
b) Herstellung von 2,3,5-Trichlortoluol
   Zu einer Mischung aus 250 g Triphenylphosphin und 250 ml Chlorbenzol wurden unter intensivem Rühren innerhalb von 2 Stunden 70 g Chlor eingeleitet, wobei die Temperatur 40°C betrug. Zu der dann vorliegenden Lösung ließ man bei 40°C die gemäß a) erhaltene Lösung innerhalb von 60 Minuten zutropfen. Die Temperatur des Reaktionsgemisches wurde nach und nach auf 200°C erhöht, wobei Chlorwasserstoff enthaltendes Chlorbenzol abdestillierte. Nach Beendigung der Gasentwicklung wurde ein Druck von 20 mbar angelegt und weiter destilliert. Das so erhaltene Destillat wurde einer erneuten fraktionierten Destillation unterworfen. Man erhielt 106 g 2,3,5-Trichlortoluol.

## Patentansprüche

1. Verfahren zur Herstellung von substituierten Chloraromaten der allgemeinen Formel (I) 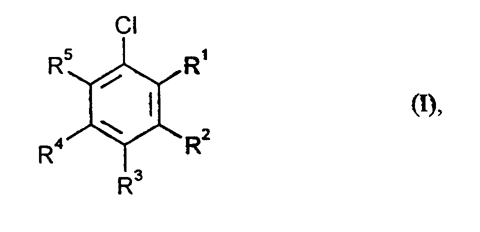 in der
R¹ bis R⁵ unabhängig voneinander jeweils Wasserstoff, C₁-C₁₀-Alkyl, gegebenenfalls substituiertes C₆-C₁₀-Aryl oder Halogen bedeuten, wobei mindestens einer dieser Reste von Wasserstoff verschieden ist,
dadurch gekennzeichnet, daß man substituierte Phenole der Formel (II) 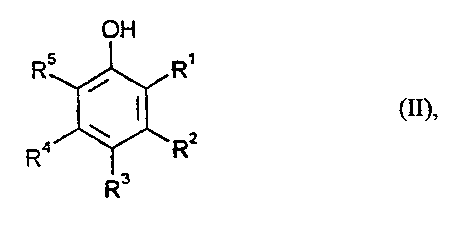 in der
R¹ bis R⁵ die bei Formel (I) angegebene Bedeutung haben,
mit Triphenylphosphindichlorid umsetzt.

2. Verfahren nach Anspruch 1, dadurch gekennzeichnet, daß in den Formeln (I) und (II) R¹ bis R⁵, unabhängig voneinander, jeweils Wasserstoff, geradkettiges oder verzweigtes C₁-C₄-Alkyl, gegebenenfalls durch C₁-C₄-Alkyl und/oder Halogen substituiertes Naphthyl oder Phenyl, Fluor oder Chlor bedeuten, wobei mindestens einer dieser Reste von Wasserstoff verschieden ist.

3. Verfahren nach Ansprüchen 1 und 2, dadurch gekennzeichnet, daß man chlorhaltige Verbindungen der Formel (II) in Form von Chlorierungs-Reaktionsgemischen einsetzt.

4. Verfahren nach Ansprüchen 1 bis 3 zur Herstellung von substituierten Chloraromaten der Formel (I), in der wenigstens einer der Reste R¹ bis R⁵ für Chlor steht, dadurch gekennzeichnet, daß man zunächst ein Phenol der Formel (II), in der mindestens 2 und höchstens 4 der Reste R¹ bis R⁵ für Wasserstoff stehen, auf übliche Weise chloriert und dann das erhaltene Reaktionsgemisch mit Triphenylphosphindichlorid umsetzt.

5. Verfahren nach Ansprüchen 1 bis 4, dadurch gekennzeichnet, daß man das Triphenylphosphindichlorid in situ erzeugt, indem man Triphenylphosphin mit Chlor oder Triphenylphosphinoxid mit Phosgen in einem inerten Lösungsmittel umsetzt.

6. Verfahren nach Ansprüchen 1 bis 5, dadurch gekennzeichnet, daß man es in Gegenwart eines inerten Lösungsmittels durchführt.

7. Verfahren nach Ansprüchen 1 bis 6, dadurch gekennzeichnet, daß man zu Triphenylphosphindichlorid die zu chlorierende Substanz der allgemeinen Formel (II) in einem Molverhältnis von 0,5 bis 10 hinzufügt.

8. Verfahren nach Ansprüchen 1 bis 7, dadurch gekennzeichnet, daß man die Reaktanten bei 0 bis 200°C zusammenfügt und die Umsetzung bei Temperaturen im Bereich von 50 bis 250°C durchführt.

9. Verfahren nach Ansprüchen 1 bis 8, dadurch gekennzeichnet, daß man das nach Beendigung der Umsetzung vorliegende Reaktionsgemisch aufarbeitet, indem man das hergestellte Produkt der Formel (I) und gegebenenfalls das inerte Lösungsmittel abdestilliert.

10. Verfahren nach Ansprüchen 1 bis 9, dadurch gekennzeichnet, daß man es in Gegenwart einers inerten Lösungsmittels durchführt und nach der Abtrennung des Lösungsmittels und des Produkts der Formel (I) den verbleibenden Rückstand in einem inerten Lösungsmittel löst, durch die Einwirkung von Phosgen daraus wieder Triphenylphosphindichlorid erzeugt und so erhaltenes Triphenylphosphindichlorid erneut für die Umsetzung mit einer Verbindung der Formel (I) einsetzt.

## Claims

1. Process for preparing substituted chloroaromatics of the general formula (I) 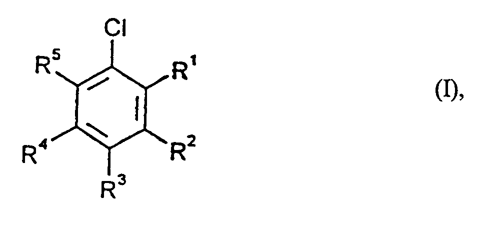 where
R¹ to R⁵ are each, independently of one another, hydrogen, C₁-C₁₀-alkyl, unsubstituted or substituted C₆-C₁₀-aryl or halogen, with at least one of these radicals being different from hydrogen,
characterized in that substituted phenols of the formula (II) 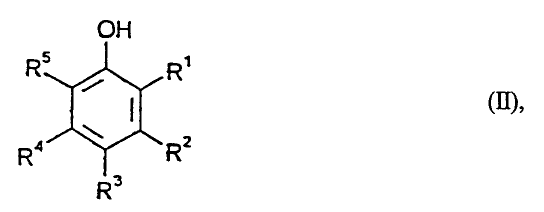 where
R¹ to R⁵ are as defined for formula (I),
are reacted with dichlorotriphenylphosphorus.

2. Process according to Claim 1, characterized in that in the formulae (I) and (II) R¹ to R⁵ are each, independently of one another, hydrogen, straight-chain or branched C₁-C₄-alkyl, unsubstituted naphthyl or phenyl or C₁-C₄-alkyl- and/or halogen-substituted naphthyl or phenyl, fluorine or chlorine, with at least one of these radicals being different from hydrogen.

3. Process according to Claim 1 or 2, characterized in that chlorine-containing compounds of the formula (II) are used in the form of chlorination reaction mixtures.

4. Process according to any of Claims 1 to 3 for preparing substituted chloroaromatics of the formula (I) in which at least one of the radicals R¹ to R⁵ represents chlorine, characterized in that a phenol of the formula (II) in which at least 2 and at most 4 of the radicals R¹ to R⁵ represent hydrogen is first chlorinated in a customary manner and the reaction mixture obtained is then reacted with dichlorotriphenylphosphorus.

5. Process according to any of Claims 1 to 4, characterized in that the dichlorotriphenylphosphorus is generated in situ by reacting triphenylphosphine with chlorine or triphenylphosphine oxide with phosgene in an inert solvent.

6. Process according to any of Claims 1 to 5, characterized in that it is carried out in the presence of an inert solvent.

7. Process according to any of Claims 1 to 6, characterized in that the substance of the general formula (II) to be chlorinated is added to dichlorotriphenylphosphorus in a molar ratio of from 0.5 to 10.

8. Process according to any of Claims 1 to 7, characterized in that the reactants are combined at from 0 to 200°C and the reaction is carried out at temperatures in the range from 50 to 250°C.

9. Process according to any of Claims 1 to 8, characterized in that the reaction mixture present after the reaction is complete is worked up by distilling off the product of the formula (I) and, if applicable, the inert solvent.

10. Process according to any of Claims 1 to 9, characterized in that it is carried out in the presence of an inert solvent and after separating off the solvent and the product of the formula (I) the residue is dissolved in an inert solvent, dichlorotriphenylphosphorus is again generated therefrom by the action of phosgene and dichlorotriphenylphosphorus thus obtained is reused for the reaction with a compound of the formula (I).

## Revendications

1. Procédé de préparation de composés aromatiques chlorés substitués de formule générale (I) : 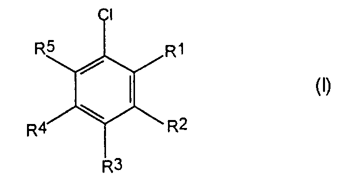 dans laquelle :
R¹ à R⁵ représentent, indépendamment l'un de l'autre, chaque fois hydrogène, alcoyle en C₁-C₁₀, aryle en C₆-C₁₀ facultativement substitué ou halogène, où au moins l'un de ces restes est différent de hydrogène,
caractérisé en ce qu'on fait réagir un phénol substitué de formule (II) : 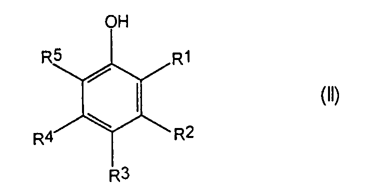 dans laquelle :
R¹ à R⁵ ont la signification donnée pour la formule (I),
avec du dichlorure de triphénylphosphine.

2. Procédé suivant la revendication 1, caractérisé en ce que, dans les formules (I) et (II), R¹ à R⁵ représentent, indépendamment l'un de l'autre, chaque fois hydrogène, alcoyle en C₁-C₄ linéaire ou ramifié, naphtyle ou phényle facultativement substitué par alcoyle en C₁-C₄ et/ou halogène, fluor ou chlore, où au moins l'un de ces restes est différent de hydrogène.

3. Procédé suivant les revendications 1 et 2, caractérisé en ce que l'on met en oeuvre des composés chlorés de formule (II) sous forme de mélanges réactionnels de chloration.

4. Procédé suivant les revendications 1 à 3, pour la préparation de composés aromatiques chlorés substitués de formule (I), dans laquelle au moins l'un des restes R¹ à R⁵ représente chlore, caractérisé en ce que l'on chlore d'abord, de manière usuelle, un phénol de formule (II), dans laquelle au moins 2 et au maximum 4 restes R¹ à R⁵ représentent hydrogène et ensuite, on fait réagir le mélange réactionnel obtenu avec le dichlorure de triphénylphosphine.

5. Procédé suivant les revendications 1 à 4, caractérisé en ce qu'on produit le dichlorure de triphénylphosphine in situ, par le fait que l'on fait réagir la triphénylphosphine avec du chlore ou l'oxyde de triphénylphosphine avec du phosgène dans un solvant inerte.

6. Procédé suivant les revendications 1 à 5, caractérisé en ce qu'on le réalise en présence d'un solvant inerte.

7. Procédé suivant les revendications 1 à 6, caractérisé en ce qu'on ajoute au dichlorure de triphénylphosphine, la substance à chlorer de formule générale (II) en un rapport molaire allant de 0,5 à 10.

8. Procédé suivant les revendications 1 à 7, caractérisé en ce qu'on rassemble les réactifs entre 0 et 200°C et qu'on réalise la réaction à des températures situées dans l'intervalle allant de 50 à 250°C.

9. Procédé suivant les revendications 1 à 8, caractérisé en ce qu'on traite le mélange réactionnel présent après achèvement de la réaction en ce que l'on sépare par distillation le produit préparé de formule (I) et facultativement, le solvant inerte.

10. Procédé suivant les revendications 1 à 9, caractérisé en ce qu'on le réalise en présence d'un solvant inerte et après la séparation du solvant et du produit de formule (I), on dissout le résidu restant dans un solvant inerte, on produit à nouveau du dichlorure de triphénylphosphine par action de phosgène et on met en oeuvre le dichlorure de triphénylphosphine ainsi à nouveau obtenu pour la réaction avec un composé de formule (I).
